# EUROPÄISCHE PATENTSCHRIFT

(11) **EP 0 450 628 B1**
(45) Veröffentlichungstag und Bekanntmachung des Hinweises auf die Patenterteilung: **16.08.1995**
(21) Anmeldenummer: 91105361.9
(22) Anmeldetag: 04.04.1991
(51) Int. Cl.: C07K 17/08, C07K 17/06, C12N 9/96, C12N 11/06

(54) **Saccharid-modifizierte, wasserlösliche Proteine**
Saccharide-modified, watersoluble proteins
Protéines solubles dans l'eau, modifiées avec des saccharides

(30) Priorität: 05.04.1990 DE 4011084
(43) Veröffentlichungstag der Anmeldung: 09.10.1991
(73) Patentinhaber: BOEHRINGER MANNHEIM GMBH, 68298 Mannheim (DE); Gesellschaft für Biotechnologische Forschung mbH (GBF), D-38124 Braunschweig (DE)
(72) Erfinder: Tischer, Wilhelm, Dr., W-8123 Peissenberg (DE); Klein, Joachim, Prof. Dr. rer. nat., W-3300 Braunschweig (DE); Müller, Rolf-Joachim, Dr. rer. nat., W-3170 Gifhorn (DE); Engelke, Stephan, Dr. rer. nat., W-4406 Drensteinfurt (DE)
(74) Vertreter: Huber, Bernhard, Dipl.-Chem.

(56) Entgegenhaltungen:
- EP-A- 222 380
- Chemical Abstracts, Band 108, Nr. 21, 23. Mai 1988, Columbus, Ohio, USA A. MATSUSHIMA et al. S. 310, Zusammenfassung Nr. 182 535g & Yuki Gosei Kagaku Kyokaishi 1988, 46(3), 232-43 (Japan)
- CHEMICAL ABSTRACTS, Band 104, Nr. 2. 13. Januar 1986, Columbus, Ohio, USA J. KLEIN et al., S. 6259, Zusammenfassung Nr. 6 251v & Makromol. Chem., Rapid Comm. 1985, 6(10), 675-8
- CHEMICAL ABSTRACTS, Band 107, Nr. 8, 24. August 1987, Columbus, Ohio J. Klein et al.
- CHEMICAL ABSTRACTS, Band 112, Nr. 10, 5. März 1990, Columbus, Ohio, USA J. KLEIN et al.
- CHEMICAL ABSTRACTS, Band 112, Nr. 12, 19. März 1990, Columbus, Ohio, USA J. KLEIN et al.
- CHEMICAL ABSTRACTS, Band 113, Nr. 2, 9. Juli 1990, Columbus, Ohio, USA J. KLEIN et al.
- CHEMICAL ABSTRACTS, Band 114, Nr. 4, 28. Januar 1991, Columbus, Ohio, USA J. KLEIN et al.

## Beschreibung

Die Erfindung betrifft Saccharid-modifizierte, wasserlösliche Proteine, ein Verfahren zu ihrer Herstellung sowie ihre Verwendung.

Es ist bekannt, Proteine zu modifizieren, um auf diese Weise bestimmte Eigenschaften wie ihre Löslichkeit, ihre Verträglichkeit bei Verwendung von hydrophoben Materialien wie z.B. bei Kunststoffen, ihre Lagerstabilität, ihre Aktivität in nicht wäßrigen Systemen, z.B. in organischen Lösungsmitteln, oder ihre biologische Halbwertszeit zu verbessern.

Aus R.D. Schmidt, Adv. Biochem. Eng. (1979) 12, 41-118 ist bekannt, daß z.B. die Stabilität, Löslichkeit oder enzymatische Aktivität von Proteinen durch eine chemische Derivatisierung mit niedermolekularen oder auch mit hochmolekularen Reagenzien sowie über eine Quervernetzung mit bi- oder polyfunktionellen Reagenzien modifiziert werden kann.

Als besonders geeignete Reagenzien haben sich bislang wasserlösliche Polysaccharide wie Dextran oder Ficoll erwiesen. So ist es z.B. aus der DE-OS 35 41 186 bekannt, auf diese Weise eine wasserlösliche Peroxidase herzustellen. Desweiteren ist aus der EP-A-0 222 308 ein Verfahren zur Stabilisierung von Creatinkinase, aus der EP-A-0 201 805 die Stabilisierung von Sarcosinoxidase und aus der EP-A-0 069 379 ein Verfahren zur Herstellung einer löslichen Leberuricase bekannt. Bei allen diesen beschriebenen Verfahren werden die Proteine kovalent an eine wasserlösliche Trägersubstanz gebunden, die üblicherweise ein Polysaccharid ist.

Polysaccharide werden jedoch leicht durch Glycohydrolasen abgebaut. Dies ist dann von Nachteil, wenn die Polysaccharidmodifizierten Proteine (Enzyme) zur enzymatischen Analyse in der klinischen Chemie eingesetzt werden sollen, da das dabei verwendete Probenmaterial derartige endogene Glycohydrolasen enthält. Außerdem können Polysaccharide von Perjodat (einem in der klinischen Chemie für Farbnachweisreaktionen häufig eingesetzten Reagenz) gespalten werden. Polysaccharide wie lösliche Stärken, Cellulosen haben durch ihre Verzweigung oder durch ihre Derivatisierung, wie sie zur Verbesserung bzw. zur Erzielung der Wasserlöslichkeit durchgeführt werden muß, häufig wenig funktionelle Gruppen für die Bindung der Proteine.

Die Erfindung hat daher zum Ziel, Proteinkonjugate bereitzustellen, welche die zuvor beschriebenen Nachteile nicht aufweisen.

Dieses Ziel wird dadurch erreicht, daß das Protein kovalent über einen Saccharidrest an ein Poly(vinylsaccharid) gebunden ist.

Es hat sich nämlich überraschenderweise gezeigt, daß sich die Stabilität und Aktivität von in einer wäßrigen Lösung gelösten Proteinen dadurch verbessern läßt, daß man das Protein mit einem Saccharidrest konjugiert, welcher wiederum kovalent mit einem Vinylrest einer Polyvinylkette als "Backbone" verbunden ist. Diese kovalente Bindung wird dabei üblicherweise über eine Ether-, Ester- oder Amidogruppe gebildet.

Die Herstellung solcher Poly(vinylsaccharide) ist bekannt und ist zusammenfassend von J. Klein et al. in Macromol. Chem. 188, 1217-1232 (1987); Macromol. Chem. Rapid Commun. 7, 621-625 (1986); und in Macromol. Chem. 189, 805-813 (1988) sowie in der Dissertationsschrift S. Engelke, TU Braunschweig FRG (1989) beschrieben, welche hiermit ausdrücklich zum Bestandteil der Anmeldung gemacht werden. Poly(vinylsaccharide) sind wasserlösliche Polymere, die mittels einer Vinylpolymerisation aus Vinylsaccharid-Monomeren erhalten werden, welche wiederum üblicherweise durch eine Kupplungsreaktion einer ungesättigten Vinylkomponente mit einem Kohlehydrat-Derivat hergestellt werden. Die Polymerisation kann sowohl kationisch als auch auf radikalischem Wege erfolgen. Katalysatoren für die radikalische Polymerisation sind z.B. Peroxide und als Katalysatoren für die kationische Polymerisation findet beispielsweise BF₃ Verwendung.

Die im erfindungsgemäßen Konjugat enthaltenen Poly(vinylsaccharide) sind z.B. aus einer Polymerisation von monomeren Vinylsacchariden wie Allyl-, Alkylallyl-, Alkoxycarbonylallyl-, Vinylether-, Acrylamid- und Methacrylamidsacchariden, als Homopolymerisate oder Copolymerisate mit z.B. Maleinsäureanhydrid, insbesondere von Acryl- und Methacrylsacchariden sowie deren Derivaten erhältlich. Vorzugsweise enthält daher das Poly(vinylsaccharid) der Erfindung eine Polymethacrylat-, Polyacrylat-, Poly(α-alkylacrylat)-, oder Poly(α-alkylmethacrylat)-Kette, wobei Alkyl hier für einen linearen Alkylrest mit 1 bis 4 C-Atomen steht. Die Durchführung von solchen Polymerisationen sind dem Fachmann bekannt und z.B. von J. Klein et al. in den zuvor genannten Druckschriften beschrieben. Das Poly(vinylsaccharid) weist vorzugsweise ein Molekulargewicht von 1.000 bis 1.000.000 Dalton, besonders bevorzugt von 400.000 bis 500.000 Dalton auf.

Das Kohlenhydrat- bzw. Saccharid-Derivat ist im erfindungsgemäßen Konjugat vorzugsweise über eine Ether-, Ester- oder Amidogruppe mit dem Kohlenwasserstoffgerüst verbunden. Dabei sind die Etherbrücken üblicherweise inert gegen chemischen und enzymatischen Angriff.

In einer bevorzugten Ausführungsform enthält das erfindungsgemäße Konjugat zwischen Saccharidderivat und Kohlenwasserstoff-backbone einen Spacer aus 1 bis 6, vorzugsweise 3 bis 5 und insbesondere aus 4 C-Atomen.

Die im erfindungsgemäßen Konjugat enthaltenen Saccharide umfassen die gesamte Gruppe der Kohlehydrate, also neben Monosacchariden auch Disaccharide, wobei jedoch Mannose sowie Sorbose und insbesondere Lactose, Glucose, Maltose und Galactose bevorzugt sind. In einer besonderen erfindungsgemäßen Ausführungsform sind die Saccharide Aminozucker und/oder Zuckerlactone. Besonders bevorzugt sind jedoch auch ringoffene Saccharide, wie Zuckeralkohole, die z.B. durch Reduktion von Saccharosen mit NaBH₄ erhältlich sind.

Besonders bevorzugte Saccharid-Derivate sind 1-Amino-1-deoxysaccharide sowie die davon abgeleiteten N-(C₁-C₄-Alkyl)Acrylat-N'-1-deoxy-saccharose-Harnstoffe und die entsprechenden Methacrylat-Derivate. Die Alkylgruppen stellen dabei einen Spacer zwischen der Harnstoffbindung und dem Vinylester dar. Besonders bevorzugt sind dabei wiederum diejenigen Derivate, die eine C₂-Alkylgruppe enthalten, wie z.B. N-Ethyl-Metylacrylato-N'-1-deoxy-Glucitol-Harnstoff, N-Ethyl methacrylato-N'-1-deoxymaltitol-Harnstoff, N-Ethyl-Methylacrylato-N'-1-deoxycellobiitol-Harnstoff sowie N-Ethylmetacrylato-N'-1-deoxylactitol-Harnstoff.

Bevorzugte Proteine sind Hexokinase, Glucose-6-phosphatdehydrogenase, Cholesterinesterase, Glucoseoxidase, Peroxidase, Lactatdehydrogenase, Glutamatdehydrogenase, Creatinamidinohydrolase, Creatinkinase, α-Glucosidase, alkalische Phosphatase, Urokinase, Cholesterinoxidase oder Sarcosinoxidase.

Das erfindungsgemäße Proteinkonjugat weist vorzugsweise ein Gewichtsverhältnis Protein zu Poly(vinylsaccharid) von 1:1 bis 1:20 auf, besonders bevorzugt ist ein Gewichtsverhältnis von 1:3 bis 1:6.

Die Erfindung betrifft auch ein Verfahren zur Herstellung von Protein-Saccharid-Konjugaten. Dabei wird erfindungsgemäß ein Poly(vinylsaccharid), vorzugsweise in wäßriger Lösung, mit dem Protein auf an sich bekannte Weise kovalent verknüpft. Die chemischen oder enzymatischen Verknüpfungsmethoden von Proteinen mit Sacchariden, insbesondere mit Polysacchariden, sind dem Fachmann bekannt und sind z.B. in der EP-A-0 222 380, EP-A-0 069 379, und in der EP-A-0 201 805 sowie in R.D. Schmidt, Adv. Biochem. Eng. (1979) 12, 41-118 beschrieben. Üblicherweise wird dabei der Kohlehydratteil des Poly(vinylsaccharids) mit einem Aktivierungsmittel wie z.B. Trichlortriazin (TCT), Bromcyan oder 1-Cyano-4-di-methyl-aminopyridinium-tetrafluoroborat (CDAP-BF4) umgesetzt, wobei das Verhältnis von Aktivierungsmittel zu Kohlehydrat vorzugsweise 1:5 bis 1:10 beträgt. Das so aktivierte Poly(vinylsaccharid) reagiert dann in wäßrigem Medium mit dem Protein unter Ausbildung einer kovalenten Bindung. Eine Zusammenfassung solcher Fixierungsverfahren ist z.B. in J. Marshall, American Chemical Society Symposium, Series 123 (1980) 125-140 und in J. Cohen und M. Wilchek, Appl. Biochem. Biotech. 9 (1984), 285-305 beschrieben.

Für das erfindungsgemäße Herstellungsverfahren verwendet man als Poly(vinylsaccharid) vorzugsweise ein Poly(acrylsaccharid), Poly(methacrylsaccharid), Poly(α-alkylacrylsaccharid) oder Poly(α-alkylmethacrylsaccharid), wobei Alkyl einen linearen Rest mit 1 bis 4 C-Atomen darstellt.

Vorzugsweise verwendet man dabei ein Poly(vinylsaccharid), welches durch Umsetzung von 1-Amino-monosacchariden oder/und -disacchariden mit einem Isocyanat, daß eine polymerisierbare Doppelbindung aufweist und Polymerisation des so erhaltenen Produktes hergestellt ist.

Im erfindungsgemäßen Verfahren ist es des weiteren bevorzugt, ein Poly(vinylsaccharid) mit einem Molekulargewicht von 1.000 bis 1.000.000 Dalton zu verwenden.

Zur Konjugatbildung ist es weiter bevorzugt, Poly(vinylsaccharid) und Protein im Verhältnis 1:1 bis 1:20, insbesondere 1:3 bis 1:6, einzusetzen.

Die erfindungsgemäß modifizierten Poly(vinylsaccharid)-Protein-Konjugate sind auch über eine längere Zeit hinweg stabil und eignen sich daher besonders zur Verwendung in klinischchemischen Reagenzien. Die Erfindung betrifft somit auch ihre Verwendung in derartigen Reagenzien.

Die Erfindung wird durch die folgenden Beispiele in Verbindung mit den Figuren näher erläutert.
- Fig. 1: zeigt hierbei die Temperaturstabilität eines erfindungsgemäßen Polyvinylsaccharid-modifizierten Enzyms in Vergleich zu Dextran-modifiziertem und nativem Enzym;
- Fig. 2: zeigt das Eintrübungsverhalten des erfindungsgemäßen Konjugats im Vergleich mit Dextran-modifiziertem und nativem Enzym, und
- Fig. 3: zeigt einen Vergleich der Trübungsstabilitäten.

### Beispiel 1

### Synthese von N-Ethyl-methacrylato-N'-1-deoxy-glucitol-Harnstoff (1)

20 g 1-Amino-1-deoxy-glucitol (1a) werden nach dem Verfahren von Klein et al. (S. Engelke Dissertation TU Braunschweig, FRG (1989)) in 100 ml Wasser gelöst und auf -3°C abgekühlt. Bei dieser Temperatur werden 10 g Isocyanatoethyl-methylacrylat (IEM, Dow Chemical) unter heftigem Rühren tropfenweise zugesetzt, wobei die Innentemperatur unterhalb 5°C gehalten wird. Nach Beendigung der Zugabe des Isocyanats wird 12 Stunden lang gerührt und auf Raumtemperatur erwärmt. Während der Reaktionszeit wird die entstehende Emulsion zu einer klaren farblosen Lösung. Danach wird die wäßrige Lösung mit Ether extrahiert, um Spuren von ungelöstem Isocyanat zu entfernen. Nach dem Lyophilisieren der Lösung wird das Rohprodukt in Methanol gelöst und aus Aceton/Ether umkristallisiert (Ausbeute 35,3 g 95 %); Schmelzpunkt 93°C.

Auf entsprechende Weise wurden N-Ethylmethacrylato-N'-1-deoxy-maltitol-Harnstoff (2), N-Methylacrylat-N'-1-deoxy-cellobiitol-Harnstoff (3) sowie N-Ethylmethacrylato-N'-1-deoxylactitol-Harnstoff (4) aus den entsprechenden 1-Amino-1-deoxy-Zuckern hergestellt.

### Beispiel 2

### Poly(Methacrylamidomaltose)/α-Glucosidase-Konjugat

500 mg Poly(Methacrylamidomaltose) (PVS-Maltose; Herstellung nach Beispiel 9, MG500.000, Staudinger-Index in 0,1 M Na₂SO₄ = 23 ml/g) werden in 20 ml Wasser gelöst und mit 200 mg 1-Cyano-4-dimethylamino-pyridinium-tetrafluoroborat (CDAP) und mit 2,4 ml Triethylamin (TEA) versetzt und mit 1 M KH₂PO₄ auf pH 8,4 eingestellt. Die auf diese Weise erhaltene aktivierte PVS-Maltose wird mit 5 ml α-Glucosidase (Boehringer Mannheim), entsprechend 200 mg Protein (gelöst in 10 mM Kaliumphosphatpuffer, pH 7,8) vereinigt und der pH wird mit 1 M KH₂PO₄ auf einen pH-Wert von 7,6 eingestellt. Dabei erhält man eine opale Lösung. Nach einer 4,5-stündigen Inkubation bei Raumtemperatur wird über Nacht gegen 10 l einer 50 mM Kaliumphosphatpuffer-Lösung, pH 7,15 dialysiert. Auf diese Weise werden 27 ml eines PVS-Maltose-Glucosidase-Konjugat-Dialysates mit 106 U/ml erhalten.

Eine FPLC von 2 mg des so erhaltenen Konjugats über Superose 6 (Pharmacia Uppsala Schweden) zeigte, daß 98 % des eingesetzten Enzyms an das Poly(vinylsaccharid) gebunden sind (Bedingungen: Druck 20 bar, Messung bei 280 nm, Retentionszeit natives Enzym 39,6, Konjugat 14,98).

### Beispiel 3 (Vergleich)

### Dextran-T40-α-Glucosidase-Konjugat

Wie in Beispiel 2 beschrieben, werden 2 g Dextran-T40 mit 20 ml Wasser, 400 mg CDAP und 4,8 ml TEA mit einer 1 M KH₂PO₄-Lösung auf pH 8,4 eingestellt. Nach Beendigung der Aktivierung werden 5 ml der aktivierten Dextranlösung mit 5 ml α-Glucosidase vereinigt. Bei einem pH von 7,6 (eingestellt mit KH₂PO₄) bleibt die Lösung klar. Auf diese Weise werden 10,5 ml eines Dextran/Glucose-Konjugat-Dialysates mit 284 U/ml erhalten.

### Beispiel 4

Wie in Beispiel 3 beschrieben, wird ein Glutamat-Dehydrogenase-Poly(vinylsaccharid)-Konjugat hergestellt.

### Beispiel 5

Wie in Beispiel 3 beschrieben, wird ein Glutamat-Dehydrogenase-Dextran T40-Konjugat hergestellt.

### Beispiel 6

An den in den Beispielen 2 und 3 hergestellten modifizierten α-Glucosidasen wird die Temperatur-Stabilität bestimmt. Dabei werden jeweils Konjugate mit einer Aktivität von 20 U/ml in einem 50 mM Kaliumphosphatpuffer, pH 7,1 bei einer Temperatur von 45°C belastet und die Aktivität alle 15 Minuten bestimmt.

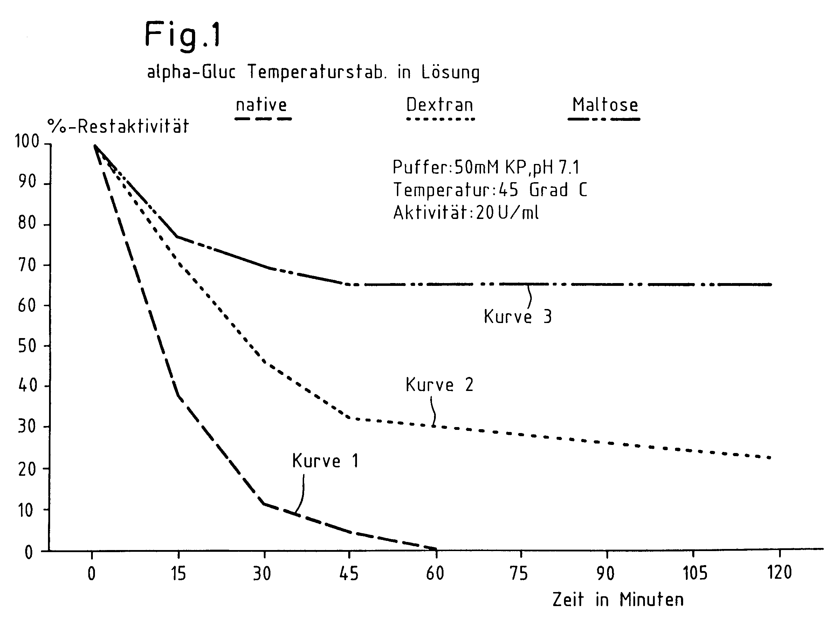

Dabei zeigt sich, daß das native Enzym schon nach 60 Minuten vollkommen inaktiviert ist und das Dextran-modifizierte Enzym nach einer zweistündigen Behandlung nur noch eine Restaktivität von etwa 20 % aufweist, wohingegen das Polyvinylmaltose-Derivat nach einer zweistündigen Behandlung noch eine Restaktivität von immerhin 65 % aufweist. Darüberhinaus zeigt das Poly(vinylsaccharid)-modifizierte Enzym nach einer 45- bis 60minütigen Behandlung bzw. Inkubation eine konstante Restaktivität, die kaum mehr weiter abnimmt. Die Ergebnisse sind in Fig. 1 dargestellt.

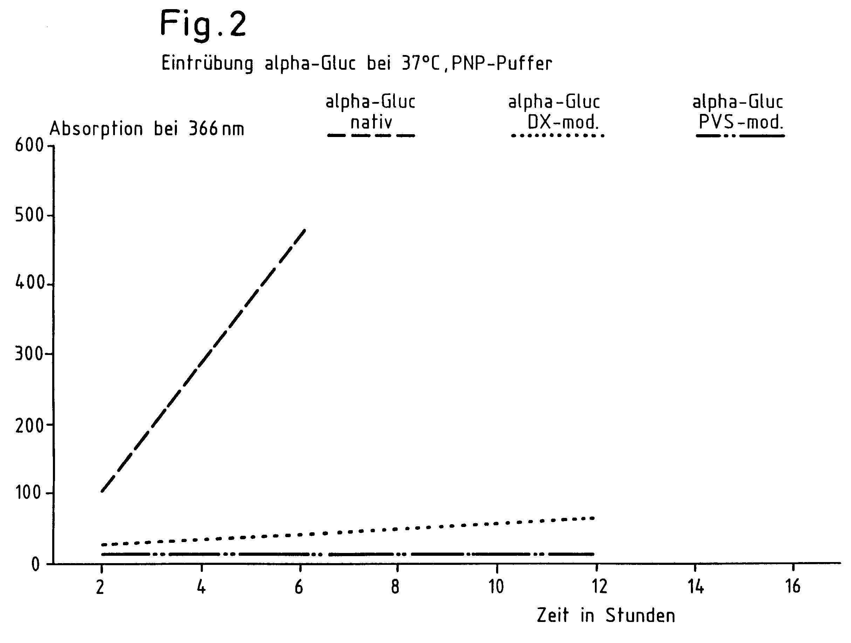

Zusätzlich wurde die Eintrübung der nativen und der modifizierten α-Glucosidase-Lösungen bei einer Inkubations-Temperatur von 37°C bestimmt. Die Ergebnisse dazu sind in Fig. 2 dargestellt. Dabei zeigt sich, daß die erfindungsgemäß modifizierte α-Glucosidase auch nach einer 12stündigen Inkubation noch keinerlei Eintrübungsmerkmale aufweist.

### Beispiel 7

### Eintrübung der Glutamat-Dehydrogenase

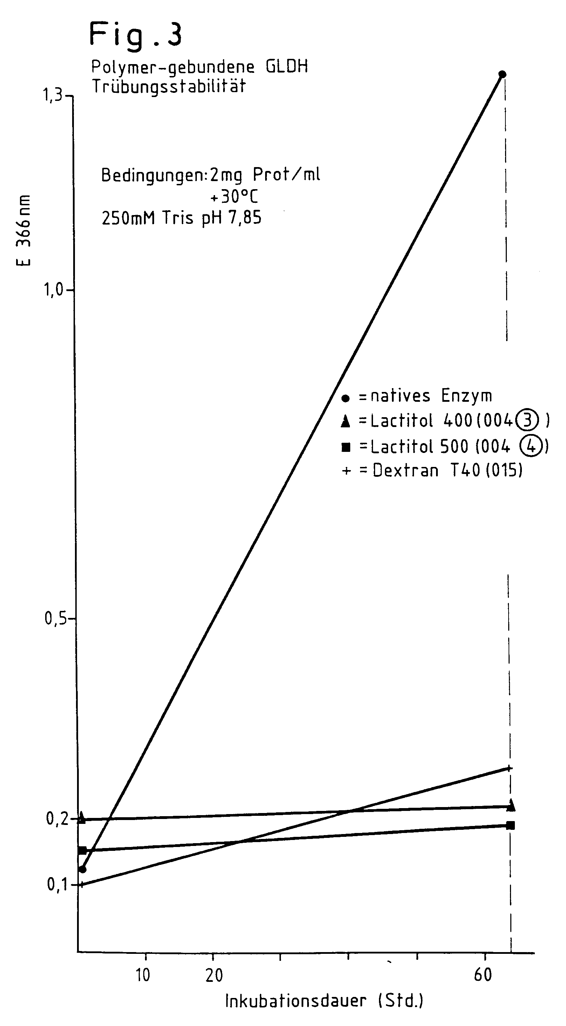

Wie in Beispiel 6 beschrieben, wird die Trübungs-Stabilität von Glutamat-Dehydrogenase bestimmt. Dabei werden 2 mg Protein pro ml bei 30°C in einem 250 mM Tris-Puffer, pH 7,85 inkubiert. Die Ergebnisse sind in Fig. 3 dargestellt. Dabei zeigte sich, daß die Trübung des erfindungsgemäß modifizierten Enzyms bei einer 64-stündigen Inkubation lediglich um etwa 25 % zunimmt, wohingegen die Trübung des nach dem Stand der Technik mit Dextran T40 modifizierten Enzyms im gleichen Zeitraum um 175 % zunimmt.

### Beispiel 8

### 1-Methacrylamido-1-desoxymaltitol

### 100 g 1 Amino-1-desoxymaltitol ≙ 0,291 mol (MG 343,3) 44,86 g Methacrylsäureanhydrid ≙ 0,291 mol (MG 154,17)

In einen 500 ml Dreihalskolben legt man 150 ml getr. Methanol bei -5°C vor und suspendiert 25 g (0,073 mol) 1-Amino-1-desoxy-maltitol (gepulvert). Man kühlt auf -10°C ab und tropft 11,26 g (0,073 mol) Methacrylsäureanhydrid langsam zu. Danach wird auf 0°C erwärmt und 24 Stunden bei dieser Temperatur gerührt. Anschließend wird 90 h bei +4°C gerührt. Bei RT wird über einen Faltenfilter filtriert und bei maximal 30°C Badtemperatur eingedampft. Das dabei erhaltene gelbe Öl wird mit 50 ml Wasser aufgenommen und 1 x mit 100 ml peroxidfreiem Ether und 3 x mit 50 ml peroxidfreiem Ether extrahiert. Am Hochvakuum wird bei max. 30°C das Wasser abgezogen und über Nacht im Exsikkator über P₂O₅ getrocknet.
Auswaage: ca. 40 g
Im Exsikkator wird weiter über P₂O₅ getrocknet:
31,31 g ≙ 104 % d. Th.

### 1 Methacrylamido-1-desoxy-maltitol

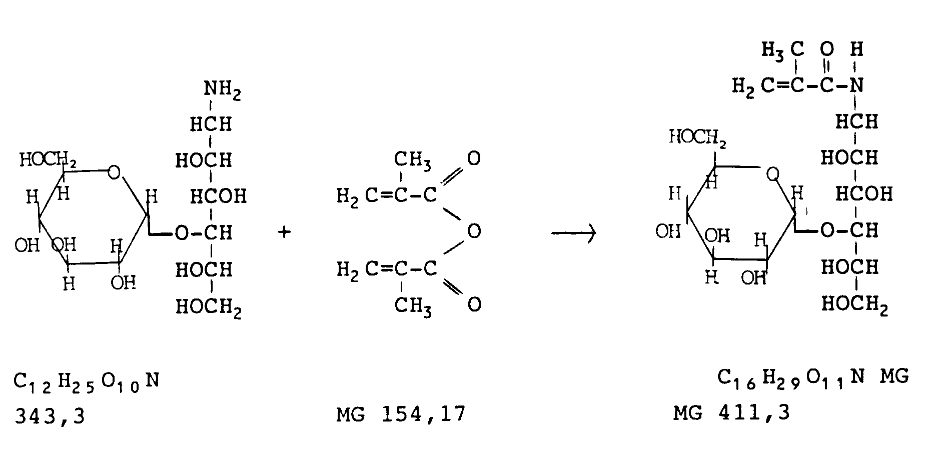

### Beispiel 9

### Polymerisation von 1-Methacrylamido-1-desoxymaltitol

20 g Monomer (H 443) ≙ 0,97 mol/l
91,2 mg (NH₄)₂S₂O₈ ≙ 8 - 10⁻³mol/l
46,3 mg Na₂S₂O₅ ≙ 4,0-10⁻³mol/l Initiatoren

| | |
|---|---|
| Volumen: | 50 ml bidest. H₂O |
| Zeit: | 4 Tage |
| Temp.: | 4°C |

Im 250 ml Dreihalskolben mit N₂-Luftballon, (Stickstoffatmosphäre) Septum und Stopfen legt man das Monomer (Herstellung nach Beispiel 8) gelöst in 40 ml bidest. H₂O vor. Es wird auf 4°C abgekühlt und es werden die Initiatoren zugegeben. Dazu werden die Salze in je 5 ml bidest. H₂O gelöst und durch das Septum zuerst das Ammoniumperoxodisulfat und danach das Natriumdisulfit eingespritzt. Nach 4 Tagen Rühren tropft man den Ansatz in 3 l Methanol ein, das 0,1 % Natriumacetat enthält. Das weiß ausgefallende Produkt wird unter Vakuum abfiltriert. Nach kurzem Trocknen löst man es in ca. 50 ml bidest. H₂O auf und fällt es nochmal durch Eintropfen in 3 l Methanol aus.
Auswaage: 6,22 g Poly(Methacrylamidomaltose)

## Patentansprüche (Patentansprüche für folgende(n) Vertragsstaat(en): AT, BE, CH, LI, DE, DK, FR, GB, GR, IT, LU, NL, SE)

1. Wasserlösliches Saccharid-modifiziertes Protein-Konjugat,
**dadurch gekennzeichnet,**
daß das Protein kovalent über einen Saccharidrest an ein Poly(vinylsaccharid) gebunden ist.

2. Protein-Konjugat nach Anspruch 1,
**dadurch gekennzeichnet,**
daß das Saccharid ein Aminozucker, ein Zuckerlacton oder ein ringoffener Zuckeralkohol ist.

3. Protein-Konjugat nach Anspruch 1 oder 2,
**dadurch gekennzeichnet,**
daß das Protein Hexokinase, Glucose-6-phosphat-dehydrogenase, Cholesterinesterase, Glucoseoxidase, Peroxidase, Lactatdehydrogenase, Glutamatdehydrogenase, Creatinamidinohydrolase, Creatinkinase, α-Glucosidase, alkalische Phosphatase, Urokinase, Cholesterinoxidase oder Sarcosinoxidase ist.

4. Protein-Konjugat nach einem der vorhergehenden Ansprüche,
**dadurch gekennzeichnet,**
daß das Poly(vinylsaccharid) eine Polymethacrylat-, Polyacrylat-, Poly(α-alkylacrylat)-, Poly(α-alkylmethacrylat)-Kette enthält, wobei Alkyl für einen linearen C₁-C₄-Alkyl-Rest steht.

5. Protein-Konjugat nach einem der vorhergehenden Ansprüche,
**dadurch gekennzeichnet,**
daß das Gewichtsverhältnis Protein zu Poly(vinylsaccharid) von 1:1 bis 1:20 beträgt.

6. Protein-Konjugat nach einem der vorhergehenden Ansprüche,
**dadurch gekennzeichnet,**
daß das Poly(vinylsaccharid) ein Molekulargewicht von 1.000 bis 1.000.000 Dalton aufweist.

7. Verfahren zur Herstellung von Saccharid-Protein-Konjugaten durch Konjugation von Saccharid und dem Protein unter Bildung einer kovalenten Bindung,
**dadurch gekennzeichnet,**
daß man als Saccharid ein Poly(vinylsaccharid) verwendet.

8. Verfahren nach Anspruch 7 ,
**dadurch gekennzeichnet,**
daß man ein Poly(vinylsaccharid) mit einem Molekulargewicht von 1.000 bis 1.000.000 Dalton verwendet.

9. Verfahren nach einem der Ansprüche 7 oder 8,
**dadurch gekennzeichnet**,
daß man zur Konjugatbildung Poly(vinylsaccharid) und Protein in einem Gewichtsverhältnis von 1:1 bis 1:20 einsetzt.

10. Verfahren nach einem der Ansprüche 7 bis 9 ,
**dadurch gekennzeichnet**,
daß man ein Poly(vinylsaccharid) verwendet, erhältlich durch Umsetzung von 1-Amino-mono- und/oder -disacchariden mit einem Isocyanat, das eine polymerisierbare Doppelbindung aufweist und das so erhaltene Produkt anschließend polymerisiert.

## Patentansprüche (Patentansprüche für folgende(n) Vertragsstaat(en): ES)

1. Verfahren zur Herstellung von Saccharid-Protein-Konjugaten durch Konjugation von Saccharid und dem Protein unter Bildung einer kovalenten Bindung,
**dadurch gekennzeichnet,**
daß man als Saccharid ein Poly(vinylsaccharid) verwendet.

2. Verfahren nach Anspruch 1,
**dadurch gekennzeichnet,**
daß man als Saccharid einen Aminozucker, ein Zuckerlacton oder einen ringoffenen Zuckeralkohol verwendet.

3. Verfahren nach Anspruch 1 oder 2,
**dadurch gekennzeichnet,**
daß man als Protein Hexokinase, Glucose-6-phosphat-dehydrogenase, Cholesterinesterase, Glucoseoxidase, Peroxidase, Lactatdehydrogenase, Glutamatdehydrogenase, Creatinamidinohydrolase, Creatinkinase, α-Glucosidase, alkalische Phosphatase, Urokinase, Cholesterinoxidase oder Sarcosinoxidase verwendet.

4. Verfahren nach einem der vorhergehenden Ansprüche,
**dadurch gekennzeichnet,**
daß das Poly(vinylsaccharid) eine Polymethacrylat-, Polyacrylat-, Poly (α-alkylacrylat)-, Poly(α-alkylmethacrylat)-Kette enthält, wobei Alkyl für einen linearen C₁-C₄-Alkyl-Rest steht.

5. Verfahren nach einem der vorhergehenden Ansprüche,
**dadurch gekennzeichnet,**
daß man ein Gewichtsverhältnis Protein zu Poly(vinylsaccharid) von 1:1 bis 1:20 anwendet.

6. Verfahren nach einem der vorhergehenden Ansprüche,
**dadurch gekennzeichnet,**
daß man ein Poly(vinylsaccharid) mit einem Molekulargewicht von 1.000 bis 1.000.000 Dalton verwendet.

7. Verfahren nach einem der vorhergehenden Ansprüche,
**dadurch gekennzeichnet,**
daß man zur Konjugatbildung Poly(vinylsaccharid) und Protein in einem Gewichtsverhältnis von 1:1 bis 1:20 einsetzt.

8. Verfahren nach einem der vorhergehenden Ansprüche,
**dadurch gekennzeichnet,**
daß man ein Poly(vinylsaccharid) verwendet, erhältlich durch Umsetzung von 1-Amino-mono- und/oder -disacchariden mit einem Isocyanat, das eine polymerisierbare Doppelbindung aufweist und das so erhaltene Produkt anschließend polymerisiert.

## Claims (Claims for the following Contracting State(s): AT, BE, CH, LI, DE, DK, FR, GB, GR, IT, LU, NL, SE)

1. Water-soluble protein conjugate modified by saccharides,
**wherein**
the protein is covalently bound to a poly(vinylsaccharide) via a saccharide residue.

2. Protein conjugate as claimed in claim 1,
**wherein**
the saccharide is an amino sugar, a sugar lactone or an open-ring sugar alcohol.

3. Protein conjugate as claimed in claim 1 or 2,
**wherein**
the protein is hexokinase, glucose-6-phosphate dehydrogenase, cholesterol esterase, glucose oxidase, peroxidase, lactate dehydrogenase, glutamate dehydrogenase, creatine amidinohydrolase, creatine kinase, α-glucosidase, alkaline phosphatase, urokinase, cholesterol oxidase or sarcosine oxidase.

4. Protein conjugate as claimed in one of the previous claims,
**wherein**
the poly(vinylsaccharide) contains a polymethacrylate, polyacrylate, poly(α-alkyl-acrylate), poly(α-alkylmethacrylate) chain in which alkyl represents a linear C₁-C₄ alkyl residue.

5. Protein conjugate as claimed in one of the previous claims,
**wherein**
the weight ratio of protein to poly(vinylsaccharide) is 1:1 to 1:20.

6. Protein conjugate as claimed in one of the previous claims,
**wherein**
the poly(vinylsaccharide) has a molecular weight of 1000 to 1,000,000 daltons.

7. Process for the production of saccharide-protein conjugates by conjugation of a saccharide and the protein with formation of a covalent bond,
**wherein**
a poly(vinylsaccharide) is used as the saccharide.

8. Process as claimed in claim 7,
**wherein**
a poly(vinylsaccharide) with a molecular weight of 1000 to 1,000,000 daltons is used.

9. Process as claimed in one of the claims 7 to 8,
**wherein**
the poly(vinylsaccharide) and protein are used in a weight ratio of 1:1 to 1:20 for the conjugate formation.

10. Process as claimed in one of the claims 7 to 9,
**wherein**
a poly(vinylsaccharide) is used which is obtainable by reacting 1-amino-mono- and/or -disaccharides with an isocyanate which has a polymerizable double bond and the product obtained in this way is subsequently polymerized.

## Claims (Claims for the following Contracting State(s): ES)

1. Process for the production of saccharide-protein conjugates by conjugation of a saccharide and the protein with formation of a covalent bond,
**wherein**
a poly(vinylsaccharide) is used as the saccharide.

2. Process as claimed in claim 1,
**wherein**
an amino sugar, a sugar lactone or an open-ring sugar alcohol is used as the saccharide.

3. Process as claimed in claim 1 or 2,
**wherein**
hexokinase, glucose-6-phosphate dehydrogenase, cholesterol esterase, glucose oxidase, peroxidase, lactate dehydrogenase, glutamate dehydrogenase, creatine amidinohydrolase, creatine kinase, α-glucosidase, alkaline phosphatase, urokinase, cholesterol oxidase or sarcosine oxidase is used as the protein.

4. Process as claimed in one of the previous claims,
**wherein**
the poly(vinylsaccharide) contains a polymethacrylate, polyacrylate, poly(α-alkyl-acrylate), poly(α-alkylmethacrylate) chain in which alkyl represents a linear C₁-C₄ alkyl residue.

5. Process as claimed in one of the previous claims,
**wherein**
a weight ratio of protein to poly(vinylsaccharide) is 1:1 to 1:20 is used.

6. Process as claimed in one of the previous claims,
**wherein**
a poly(vinylsaccharide) with a molecular weight of 1000 to 1,000,000 daltons is used.

7. Process as claimed in one of the previous claims,
**wherein**
the poly(vinylsaccharide) and protein are used in a weight ratio of 1:1 to 1:20 for the conjugate formation.

8. Process as claimed in one of the previous claims,
**wherein**
a poly(vinylsaccharide) is used which is obtainable by reacting 1-amino-mono- and/or -disaccharides with an isocyanate which has a polymerizable double bond and the product obtained in this way is subsequently polymerized.

## Revendications (Revendications pour l'(les) Etat(s) contractant(s) suivant(s): AT, BE, CH, LI, DE, DK, FR, GB, GR, IT, LU, NL, SE)

1. Conjugué soluble dans l'eau d'une protéine modifiée avec un saccharide, caractérisé en ce que la protéine est liée par covalence à un poly(vinylsaccharide) par l'intermédiaire d'un reste saccharide.

2. Conjugué de protéine selon la revendication 1, caractérisé en ce que le saccharide est un sucre aminé, une lactone de sucre ou un alcool de sucre à chaîne ouverte.

3. Conjugué de protéine selon la revendication 1 ou 2, caractérisé en ce que la protéine est une hexokinase, une glucose-6-phosphate-déshydrogénase, une cholestérol-estérase, une glucose-oxydase, une peroxydase, une lactate-déshydrogénase, une glutamate-déshydrogénase, une créatine-amidinohydrolase, une créatine-kinase, une α-glucosidase, une phosphatase alcaline, une urokinase, une cholestérol-oxydase ou une sarcosine-oxydase.

4. Conjugué de protéine selon l'une des revendications précédentes, caractérisé en ce que le poly(vinylsaccharide) contient une chaîne de polyméthacrylate, de polyacrylate, de poly(α-alkylacrylate), de poly(α-alkylméthacrylate), "alkyl" représentant un reste alkyle linéaire en C₁-C₄.

5. Conjugué de protéine selon l'une des revendications précédentes, caractérisé en ce que le rapport en masse de la protéine au poly(vinylsaccharide) est compris entre 1:1 et 1:20.

6. Conjugué de protéine selon l'une des revendications précédentes, caractérisé en ce que le poly(vinylsaccharide) a une masse moléculaire de 1 000 à 1 000 0000 daltons.

7. Procédé de préparation de conjugués saccharide-protéine par conjugaison du saccharide et de la protéine avec formation d'une liaison covalente, caractérisé en ce que l'on utilise comme saccharide un poly(vinylsaccharide).

8. Procédé selon la revendication 7, caractérisé en ce que l'on utilise un poly(vinylsaccharide) ayant une masse moléculaire de 1 000 à 1 000 000 daltons.

9. Procédé selon l'une des revendications 7 ou 8, caractérisé en ce que l'on utilise pour la formation du conjugué le poly(vinylsaccharide) et la protéine en un rapport en masse de 1:1 à 1:20.

10. Procédé selon l'une des revendications 7 à 9, caractérisé en ce que l'on utilise un poly(vinylsaccharide) que l'on peut obtenir par réaction de 1-amino-mono- et/ou -disaccharides avec un isocyanate qui comporte une double liaison polymérisable, puis polymérisation du produit ainsi obtenu.

## Revendications (Revendications pour l'(les) Etat(s) contractant(s) suivant(s): ES)

1. Procédé de préparation de conjugués saccharide-protéine par conjugaison du saccharide et de la protéine avec formation d'une liaison covalente, caractérisé en ce que l'on utilise comme saccharide un poly(vinylsaccharide).

2. Procédé selon la revendication 1, caractérisé en ce que l'on utilise comme saccharide un sucre aminé, une lactone de sucre ou un alcool de sucre à chaîne ouverte.

3. Procédé selon la revendication 1 ou 2, caractérisé en ce que l'on utilise comme protéine une hexokinase, une glucose-6-phosphate-déshydrogénase, une cholestérol-estérase, une glucose-oxydase, une peroxydase, une lactate-déshydrogénase, une glutamate-déshydrogénase, une créatine-amidinohydrolase, une créatine-kinase, une α-glucosidase, une phosphatase alcaline, une urokinase, une cholestérol-oxydase ou une sarcosine-oxydase.

4. Procédé selon l'une des revendications précédentes, caractérisé en ce que le poly(vinylsaccharide) contient une chaîne de polyméthacrylate, de polyacrylate, de poly(α-alkylacrylate), de poly(α-alkylméthacrylate), "alkyl" représentant un reste alkyle linéaire en C₁-C₄.

5. Procédé selon l'une des revendications précédentes, caractérisé en ce que l'on utilise un rapport en masse de la protéine au poly(vinylsaccharide) compris entre 1:1 et 1:20.

6. Procédé selon l'une des revendications précédentes, caractérisé en ce que l'on utilise un poly(vinylsaccharide) ayant une masse moléculaire de 1 000 à 1 000 0000 daltons.

7. Procédé selon l'une des revendications précédentes, caractérisé en ce que l'on utilise pour la formation du conjugué le poly(vinylsaccharide) et la protéine en un rapport en masse de 1:1 à 1:20.

8. Procédé selon l'une des revendications précédentes, caractérisé en ce que l'on utilise un poly(vinylsaccharide) que l'on peut obtenir par réaction de 1-amino-mono- et/ou -disaccharides avec un isocyanate qui comporte une double liaison polymérisable, puis polymérisation du produit ainsi obtenu.
